# EUROPEAN PATENT APPLICATION

(11) **EP 2 067 469 A1**
(43) Date of publication of application: **10.06.2009**
(21) Application number: 08253639.2
(22) Date of filing: 06.11.2008
(51) Int. Cl.: A61K 9/16, A61K 31/495, A61K 31/787

(54) **Chewable formulations**

(30) Priority: 06.11.2007 US 985836 P
(71) Applicant: Teva Pharmaceutical Industries Ltd., 49131 Petah Tiqva (IL)
(72) Inventor: Cohen, Rakefet, Zur-ygal Kokhav-yair (IL)
(74) Representative: Wong, Kit Yee

(57) **Abstract**

The invention encompasses a solid dose pharmaceutical composition comprising a disagreeable-tasting drug and processes for preparation of the pharmaceutical composition. The pharmaceutical composition is designed not to be swallowed immediately.

## Description

### CROSS-REFERENCE TO RELATED APPLICATIONS

This application claims the benefit of U.S. Provisional Application No. 60/985,836, filed November 6, 2007, hereby incorporated by reference.

### FIELD OF THE INVENTION

The invention encompasses a solid dose pharmaceutical composition comprising a disagreeable-tasting drug and processes for preparation of the pharmaceutical composition. In particular, the pharmaceutical composition is not designed to be swallowed immediately.

### BACKGROUND OF THE INVENTION

The chemical name of cetirizine is (±)-[2-[4-[(4-chlorophenyl)phenylmethyl] -1-piperazinyl] ethoxy]acetic acid, having the following structural formula I:

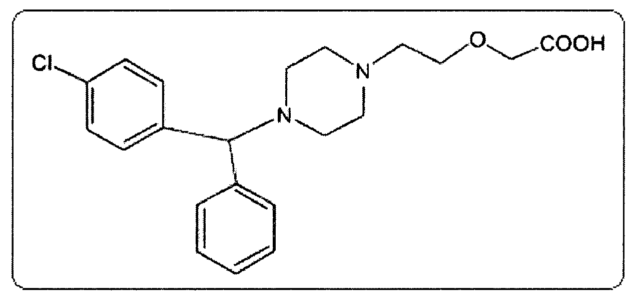

Cetirizine is an orally active, selective H₁- receptor antagonist and useful as a non-sedating antihistamine. The commercially available products of cetirizine are film-coated, immediate release oral tablets and chewable tablets, either containing 5 mg or 10 mg of cetirizine dihydrochloride under the brand name ZYRTEC^{®}. Cetirizine hydrochloride is used for the symptomatic relief of allergic conditions and is bitter in taste.

The bitter after taste of many drugs which are orally administered often contributes to patient non-compliance in taking medicine. Patients prescribed tablets with a disagreeable taste often have difficulty swallowing such tablets, and sometimes they avoid taking their medication completely. Patient noncompliance results in patients not receiving the optimal dosage of their medication.

The art of taste masking is relatively complex and involves many variables and factors, which are case specific. There are a variety of methods utilized in taste masking. These include the use of flavour enhancers, polymer coatings, inclusion complex formations with cyclodextrin, ion exchange resins, solubility limiting methods, liposomes, and multiple emulsions, etc. Some taste masking strategies, while effective under certain instances, can adversely affect dissolution times in a patient's mouth and/or stomach. Such strategies therefore might not suit the demands of rapidly dissolvable in-mouth oral dosage forms. For example, use of flavour enhancers is not applicable for oral administration of extremely bitter tasting drugs. Inclusion complex formation with cyclodextrin is limited in use because if the association constant of the inclusion complex is too large, there is a risk that the active ingredient will not be released easily enough to allow good absorption in the gastrointestinal tract. Drug coatings using polymeric materials, such as water soluble or insoluble celluloses, lipids, sugar, and the use of sweeteners such as sorbitol, sodium saccharine, and the like, lose their taste masking effectiveness over time. Specifically, taste masking using a water soluble sugar coating often provides a bitter taste once the sugar coating dissolves in the mouth. Moreover, the use of water insoluble coatings frequently affects the release profile of the drug, thereby affecting its bioavailability.

U.S. Patent No. 6,455,533 purportedly discloses a solid pharmaceutical composition for oral administration comprising a mixture of an active substance belonging to the substituted benzhydrylpiperazine family, an optically active isomer thereof or a pharmaceutically active salt thereof and at least one cyclodextrin, wherein the mixture does not contain any inclusion complexes.

WO 05/055986 purportedly discloses a taste masked pharmaceutical composition having a bitter drug and a pH sensitive polymer, which maintain the drug in substantially amorphous form and enhances bioavailability.

WO 02/096392 purportedly discloses a taste-masked formulation comprising taste-masked particles. The taste-masked particles include an active ingredient and a taste-masking layer surrounding the active ingredient. In particular, the taste-masking layer includes a water soluble polymer and a water insoluble polymer.

WO 06/061700 purportedly discloses a resinate of cetirizine or its pharmaceutically acceptable salts or its enantiomers or pharmaceutically acceptable salts of its enantiomers such as levocetirizine dihydrochloride.

WO 05/097064 purportedly discloses a pharmaceutical composition having multiparticulates comprising a drug, a matrix material, and swelling agent. In one aspect, the multiparticulates comprise a core comprising a drug and a coating surrounding the core. The core is selected from the group consisting of a water permeable substantially drug-impermeable coating, and an anti-enteric coating.

U.S. Publication No. 2007/0086974 purportedly discloses stable and palatable taste masked pharmaceutical compositions of substituted benzhydrylpiperazines and processes for preparing them. U.S. Publication No. 2007/0086974 also suggests avoiding contacting cetirizine with alcohols having a molecular weight less than 100 as it results in a reaction between cetirizine and the alcohol causing esterification, and thereby leading to instability of the dosage form.

Therefore, a pharmaceutical composition which masks the disagreeable bitter taste of active pharmaceutical ingredients without affecting its efficacy and while stabilizing the formulation would be a significant improvement in the field of taste masking. Such compositions and the need to provide a simple process of formulating this pharmaceutical composition are addressed by this invention.

### SUMMARY OF THE INVENTION

In one embodiment, the invention encompasses a pharmaceutical composition comprising a granulate, wherein the granulate comprises a disagreeable-tasting drug, a filler and a binder, wherein the filler is low substituted hydroxypropyl cellulose, crospovidone or microcrystalline cellulose, the binder is a polymethacrylate, polyvinylpyrrolidone, copovidone, cellulose derivatives, starch, dextrin or a combination thereof, wherein the pharmaceutical composition further comprises at least one extragranular excipient, and wherein the pharmaceutical composition is designed not to be swallowed immediately.

In another embodiment, the invention encompasses a pharmaceutical composition comprising a granulate, wherein the granulate comprises cetirizine, a filler and a binder, wherein the filler is low substituted hydroxypropyl cellulose, crospovidone or microcrystalline cellulose, the binder is a polymethacrylate, polyvinylpyrrolidone, copovidone, cellulose derivatives, starch, dextrin or a combination thereof, wherein the pharmaceutical composition further comprises at least one extragranular excipient, and wherein the pharmaceutical composition is designed not to be swallowed immediately

Yet another embodiment of the invention encompasses a process for preparing a pharmaceutical composition comprising dissolving a disagreeable-tasting drug and a binder with a solvent to form a solution; admixing a filler to the solution; removing the solution to obtain a mixture; and granulating the mixture, wherein the filler is low substituted hydroxylpropyl cellulose, crospovidone or microcrystalline cellulose, the binder is a polymethacrylate, polyvinylpyrrolidone, copovidone, cellulose derivatives, starch, dextrin or a combination thereof, and wherein the pharmaceutical composition is designed not to be swallowed immediately. Preferably, the process further comprises adding extra granular excipients to obtain a final blend and compressing the final blend into a solid dosage form. The present invention further encompasses a pharmaceutical composition prepared by such a process.

One embodiment of the invention encompasses a process for preparing a pharmaceutical composition comprising dissolving cetirizine and a binder with a solvent to form a solution; admixing a filler to the solution; removing the solution to obtain a mixture; and granulating the mixture, wherein the filler is low substituted hydroxylpropyl cellulose, crospovidone or microcrystalline cellulose, the binder is a polymethacrylate, polyvinylpyrrolidone, copovidone, cellulose derivatives, starch, dextrin or a combination thereof, and wherein the pharmaceutical composition is not designed to be swallowed immediately. Preferably, the process further comprises adding extra granular excipients to obtain a final blend and compressing the final blend into a solid dosage form. The present invention further encompasses a pharmaceutical composition prepared by such a process.

### DETAILED DESCRIPTION OF THE INVENTION

The pharmaceutical compositions of the present invention and those formed according to the process of the invention are stable and palatable. Unlike some of the prior-art compositions, the pharmaceutical compositions of the present invention do not reduce the bioavailability of the drug while taste-masking a disagreeable tasting drug without using a coating. The pharmaceutical compositions of the present invention are designed not to be swallowed immediately, i.e. the pharmaceutical compositions of the present invention are preferably orodispersible or orally disintegratable. Thus, the compositions of the present invention are dosage forms that are designed to dissolve or disintegrate in the oral or buccal cavity. Examples of these include sublingual tablets, troches, buccal dosage forms, chewable tablets and orodispersible tablets (ODT).

The invention encompasses a pharmaceutical composition comprising a granulate, wherein the granulate comprises a disagreeable-tasting drug, a filler and a binder, wherein the filler is low substituted hydroxypropyl cellulose, crospovidone or microcrystalline cellulose, the binder is a polymethacrylate, polyvinylpyrrolidone, copovidone, cellulose derivatives, starch, dextrin and a combination thereof, wherein the pharmaceutical composition further comprises at least one extra-granular excipient, and wherein the pharmaceutical composition is designed not to be swallowed immediately. Preferably, the filler is not used as ion exchange resin.

Preferably, in any embodiment of the present invention, the granulate consists essentially of the disagreeable-tasting drug, filler and binder. More preferably, in any embodiment of the present invention, the granulate consists only of the disagreeable-tasting drug, filler and binder.

Preferably, the binder in any of the compositions and processes of the present invention is selected from the group consisting of a polymethacrylate, polyvinylpyrrolidone, copovidone and starch, or a combination thereof.

As used herein the term "designed not to be swallowed immediately" means that the pharmaceutical composition is intended for administration not by swallowing whole. Typically the pharmaceutical composition is administered by dissolution or disintegration in the oral cavity. Such compositions include chewable tablets, sublingual tablets, troches, buccal dosage forms, and orodispersible tablets. Preferably, the composition is in the form of a chewable tablet or an orally dispersible tablet (ODT).

Preferably, the pharmaceutical compositions of any embodiment of the present invention are retained within the oral or buccal cavity for about 2 to about 60 seconds, preferably about 3 to about 55 seconds, more preferably about 5 to about 50 seconds. Preferably for chewable tablets, the composition is retained in the oral or buccal cavity for a period of about 10 seconds or less, preferably less than about 10 seconds (e.g. from about 1 to about 9 seconds, more preferably from about 2 to about 7 seconds and most preferably from about 2 to about 6 seconds), more preferably, in the case of chewable tablets, the composition is retained in the oral or buccal cavity for a period of about 5 seconds or less, preferably less than about 5 seconds (e.g. from about 1 second to about 5 seconds, preferably from about 1 to about 4 seconds, and most preferably about 2 to about 4 seconds) before being swallowed. Preferably, in the case of orally dispersable tablets [ODT] the composition is retained in the oral or buccal cavity for about 45 seconds or less, preferably less than 45 seconds (e.g. from about 5 to about 45 seconds, more preferably from about 8 to about 45 seconds, and most preferably from about 8 to about 40 seconds).

The disagreeable-tasting drug for use in the compositions and methods of the present invention can be any drug that has an unpleasant (e.g. bitter, etc) taste. Particularly suitable for use in the compositions and methods of the present invention are orally active H₁ receptor antagonists which are useful non-sedating antihistamines, for example benzhydrylpiperazine histamine H₁ receptor antagonists. These include cetirizine, efletirizine, buclizine, etodroxizine, hydroxyzine and chlorcyclizine as well as their optically active isomers, hydrates and salts thereof. Cetirizine, and its optically active isomers and hydrates and salts thereof are particularly preferred.

In one particular embodiment, the pharmaceutical composition comprises cetirizine as the disagreeable-tasting drug. Preferably in this embodiment, the intra-granular filler is not mannitol, sorbitol, or a hydroxylated sugar. Further, in this and other embodiments of present invention the composition preferably contains about 2% or less (e.g. about 0.005% to about 2%, more preferably about 0.005% to about 1.5%, most preferably about 0.01% to about 1.5%) of impurities/degradation products of cetirizine, after storage for 3 months at 40°C and at a relative humidity of 75%. Typically the composition has less than about 1.4%, and preferably, less than about 1.4% to about 0.005% impurities/degradation products of cetirizine after storage for 3 months at 40°C and a relative humidity of 75%.

Preferably, when the disagreeable-tasting drug is cetirizine, the composition has 1.0% or less (e.g. about 0.005% to about 1.0%, preferably about 0.005% to about 0.8%, more preferably about 0.01% to about 0.2%), more preferably less than about 1.0% (e.g. about e.g. about 0.005% to about 0.9%, preferably about 0.005% to about 0.8%, more preferably about 0.01 % to about 0.5%) preferably less than about 1.0% to 0.005% impurities/degradation products of the drug after storage for 3 months at 40°C and a relative humidity of 75%. More preferably, when the disagreeable-tasting drug is cetirizine, the composition has 0.8% or less (e.g. about 0.005% to about 0.8%, more preferably about 0.005% to about 0.7% and most preferably about 0.01% to about 0.5%), preferably less than about 0.8%, (e.g. about 0.005% to about 0.7%, more preferably about 0.01% to about 0.7%), preferably less than about 0.8% to 0.005% impurities/degradation products of the drug after storage for 3 months at 40°C and a relative humidity of 75%. The above percentages may be by wt% relative to the active agent in the composition. Preferably, when referring to the active agent, preferably cetirizine, the percent impurity/degradation product is determined by HPLC area % as described below in the example section.

Typically, in any of the compositions of the present invention the disagreeable tasting drug comprises an active pharmaceutical ingredient ("API") that is a substituted benzhydrylpiperazine selected from the group consisting of cetirizine, efletirizine, buclizine, etodroxizine, hydroxyzine, and chlorcyclizine or an optically active isomer thereof, a hydrate thereof, or a pharmaceutically acceptable salt thereof. Preferably, the disagreeable tasting drug is cetirizine or a salt thereof. Optionally, the pharmaceutical composition of the present invention comprises a combination of two or more API drugs, wherein at least one of the API drugs has disagreeable taste.

Typically, in any embodiment of the present invention, the weight ratio between the filler and the disagreeable tasting drug is about 20:1 to about 3:1. Preferably, the weight ratio is about 15:1 to about 3:1. More preferably, the weight ratio is about 12:1 to about 3:1. Most preferably, the weight ratio between the filler and the disagreeable tasting drug is 10:1.

Preferably, in any of the compositions of the present invention, the weight ratio of filler to drug in the granulate is about 30:1 to about 2:1, preferably about 25:1 to about 2:1, more preferably about 20:1 to about 1:1, more preferably about 18:1 to about 2:1, most preferably about 15:1 to about 3:1. Particularly preferred in the granulate is weight ratio of filler and drug of about 12:1 to about 5:1, especially about 12:1 to about 8:1. An especially preferred weight ratio of filler to drug in the granulate is about 10:1.

Generally, in any embodiment of the present invention, the binder is at least one hydrophilic polymer or water-soluble polymer. Preferably, the binder is a water-soluble polymer. Preferably, the binder is selected from the group consisting of polymethacrylates, polyvinylpyrrolidone (PVP), copovidone, cellulose derivatives, starch, and dextrin. Preferred binders are selected from the group consisting of polymethacrylates, PVP, copovidone, cellulose derivatives and starch. Especially preferred binders include polymethacrylates, PVT, copovidone and starch. More preferably, the binder is a polymethacrylate or polyvinylpyrrolidone. Polymethacrylates include commercially available polymers such as methyl methacrylate methacrylic acid copolymers including e.g. Eudragit® E100.

Typically, in any embodiment of the present invention, the weight ratio between the binder and the disagreeable tasting drug is about 5:1 to about 1:1. Preferably, the weight ratio is about 4:1 to about 2.5:1. More preferably, the weight ratio is about 3:1 to about 1:1.

Preferably, in any embodiment of the present invention the weight ratio between the binder and the drug in the granulate is about 8:1 to about 1:1, preferably about 5:1 to about 1:1. Preferably, the weight ratio of the binder to the drug in the granulate is about 4:1 to about 2.5:1. More preferably, the weight ratio of the binder to the drug in the granulate is about 3:1 to about 1:1. The pharmaceutical compositions of any embodiment of the present invention may also contain additional excipients including, but not limited to, disintegrants, lubricants, flavors, colorants, additional fillers, binders, or glidants.

The pharmaceutical composition described above may be formed into a solid dosage form such as tablet, pill, or chewable tablet. Preferably, the composition is in the form of a chewable tablet, and may be an orodispersible or orally dissolvable tablet,

Preferably, in any embodiment of the present invention, the compositions achieve a taste-masking (i.e. the disagreeable tasting drug is rendered palatable in the composition) of the disagreeable tasting drug without the need for taste-mask coatings. More preferably, the disagreeable tasting drug is rendered palatable in the compositions of the invention without the need for flavour enhancers, polymer coatings, inclusion complex formations ion exchange resins, solubility limiting methods, liposomes and multiple emulsions. More preferably, the compositions of the present invention, and specifically the disagreeable tasting drug is rendered palatable by the provision of a granulate comprising the disagreeable tasting drug, binder and filler as described in any of the above embodiments. Most preferably, the granulates consisting essentially of the disagreeable tasting drug, binder and filler) of the compositions of present invention enable the formulation of the disagreeable tasting drug to be palatable.

The invention also encompasses a process for preparing a pharmaceutical composition according to any embodiment of the present invention, comprising dissolving a disagreeable-tasting drug and a binder with a solvent to form a solution, admixing a filler to the solution, removing the solution to obtain a mixture, and granulating the mixture, wherein the filler is low substituted hydroxypropyl cellulose, crospovidone or microcrystalline cellulose, the binder is a polymethacrylate, polyvinylpyrrolidone, copovidone, cellulose derivatives, starch, dextrin, or a combination thereof, and the pharmaceutical composition is designed not to be swallowed immediately. The preferred drug, binders and fillers are discussed above in relation to the pharmaceutical composition embodiments of the present invention, and are applicable to the above process.

Preferably the pharmaceutical composition is in the form of a chewable tablet, an orodispersible tablet or an orally disintegrating tablet. More preferably, the pharmaceutical composition is in the form of a chewable tablet or an orodispersible tablet.

Preferably, the process further comprises adding extra granular excipients to obtain a final blend and the final blend is preferably further compressed into a solid dosage form. Suitable solid dosage forms include, but are not limited to, tablets, capsules, powders, and sachets. More preferably, the granulate is compressed into a chewable tabletor into an orally dispersable tablet.

When the disagreeable-tasting drug is cetirizine, the granulate preferably has 1.4% or less (e.g. about 0.005% to about 1.4%, more preferably about 0.005% to about 1.2%, and most preferably about 0.01 % to about 1.0%) of impurities/degradation products of cetirizine after storage for 3 months at 40°C and a relative humidity of 75%. Preferably the granulate has less than about 1.4%, preferably less than about 1.4% to 0.005% impurities/degradation products of cetirizine after storage for 3 months at 40°C and a relative humidity of 75%. Preferably, when the disagreeable-tasting drug is cetirizine, the granulate has about 1.0% or less (e.g. about 0.005% to about 1.0%, preferably about 0.005% to about 0.8% and more preferably about 0.01 % to about 0.08%), and more preferably the granulate has less than about 1.0%, preferably less than about 1.0% to 0.005% impurities/degradation products of the drug after storage for 3 months at 40°C and a relative humidity of 75%. More preferably, when the disagreeable-tasting drug is cetirizine, the granulate has less than about 0.8% , preferably less than about 0.8% to 0.005% impurities/degradation products of the drug after storage for 3 months at 40°C and a relative humidity of 75%.

The invention further encompasses a process comprising admixing a filler with a disagreeable-tasting drug and admixing a binder solution to obtain to obtain a mixture, and granulating the mixture, wherein the filler is low substituted hydroxypropyl cellulose, crospovidone or microcrystalline cellulose, the binder is a polymethacrylate, polyvinylpyrrolidone, copovidone, cellulose derivatives, starch, dextrin, or a combination thereof, and the pharmaceutical composition is designed not to be swallowed immediately. The drug, binder and filler (and preferred drug, binder and filler) can be any of those discussed for any of the compositions of the invention. Preferably, the process further comprises adding extra granular excipients to obtain a final blend and the final blend is preferably further compressed into a solid dosage form. Suitable solid dosage forms include, but are not limited to, tablets, capsules, powders, and sachets. More preferably, the granulate is compressed into a chewable tablet or into oral dispersable tablet.

Any granulation method known in the art may be used to granulate the filler, for example, via dry granulation or wet granulation. Preferably, the filler and the solution of disagreeable-tasting drug and the binder are combined by wet granulation.

In most cases, the sequence for adding the components when forming the solution of the disagreeable tasting drug and the binder is unimportant, e.g. mixing first the drug with a solvent and then adding the binder; mixing first the solvent with the binder and then adding the drug; adding a solvent to a mixture of drug and binder; or adding all three components at the same time.

Suitable solvents include both aqueous and organic solvents, for example, mixtures of water and organic solvents. Suitable organic solvents include, but are not limited to, alcohols, such as ethanol, and isopropanol and ketones, such as acetone. Preferably, the solvent is an aqueous solution. More preferably, the solvent is water.

Typically, after wet granulation, the solvent is removed from the solution, via a solvent removal operation. As used herein, the term "solvent removal" when referring to wet granulation means separating the solution into at least two phases (liquid/liquid or liquid/gas) where one phase contains mostly drug and the binder, while a second phase contains most or all of the solvent. Typically, the solvent is removed via evaporation. One of the alternatives to evaporation is using an anti-solvent, a component that when added to the solution leads to precipitation of the other solutes. A suitable anti-solvent is miscible with the solvent and is also a poor solvent to the solutes. In some cases, ethanol acts as a suitable anti-solvent.

The invention further encompasses a process for preparing a chewable pharmaceutical composition comprising mixing a disagreeable-tasting drug with a binder in a solvent; admixing a filler to form a mixture; granulating the mixture, and removing the solvent to form a granulated drug-binder-filler mixture, wherein the filler is not used as an ion exchange resin. The granulated drug-binder-filler mixture may then be incorporated into a pharmaceutical composition in a process further comprising admixing at least one extra granular excipient using conventional methods such as granulation and tabletting. In any process of the present invention, the drug, binder and filler and preferred drug, binder and filler, can be any of those discussed for the compositions of the present invention.

Optionally, the drug-binder-filler mixture may be formed into a film or other form that is easily milled. The filler is selected from the group consisting of low substituted hydroxypropyl cellulose, crospovidone, or microcrystalline cellulose.

In one particular embodiment of the process when cetirizine is the disagreeable-tasting drug, the granulated drug-binder-filler mixture has less than about 1.4% to 0.005% impurities/degradation products of cetirizine after storage for 3 months at 40°C and a relative humidity of 75%. Preferably, when the disagreeable-tasting drug is cetirizine, the granulated drug-binder mixture has less than about 1.0% to 0.005% impurities/degradation products of the drug after storage for 3 months at 40°C and a relative humidity of 75%. More preferably, when the disagreeable-tasting drug is cetirizine, the granulated drug-binder mixture has less than about 0.8% to 0.005% impurities/degradation products of the drug after storage for 3 months at 40°C and a relative humidity of 75%.

Alternatively, the solvent is either partially or entirely removed from the drug-binder-filler mixture and the remaining drug-binder-filler mixture is incorporated into a pharmaceutical composition by granulation. Subsequently, the granulate is used to form tablets using any known methods in the art, for example, by compression.

The pharmaceutical composition according to the present invention can be in a various orally administrable forms. In particular, the pharmaceutical composition according to the present invention can be in the form of chewable tablets, oral dispersible tablets, oral dissolvable tablets or granules. Preferably, the pharmaceutical composition of the present invention and/or the pharmaceutical composition formed according to the processes of the present invention are chewable tablets or oral dispersible tablets. The chewable tablets of the present invention are palatable and lack the disagreeable taste, even in the absence of protecting layer/coating.

Having described the invention with reference to certain preferred embodiments, other embodiments will become apparent to one skilled in the art from consideration of the specification. The invention is further defined by reference to the following examples describing in detail the pharmaceutical compositions and processes for preparing the pharmaceutical compositions of the invention. It will be apparent to those skilled in the art that many modifications, both to materials and methods, may be practiced without departing from the scope of the invention.

### EXAMPLES

### Example 1

To cetirizine hydrochloride (5.0 mg/tablet, 2.2% w/w) in purified water (q.s.), Eudragit E100 (6 mg/tablet, 2.7% w/w) was added. Low substituted hydroxypropyl cellulose (HPC) (50 mg/tablet, 22.2% w/w) was transferred to a high shear mixer, and the solution of cetirizine HCl and Eudragit E100 was slowly added to the low substituted HPC while mixing to obtain an uniform granulate which was milled, dried in a fluid bed dryer and sieved. Artificial cherry flavor (1 mg/tablet, 0.44% w/w) was mixed with a portion of microcrystalline cellulose and then sieved with the remaining portion of microcrystalline cellulose (total to 60 mg/tablet, 26.7% w/w) and sodium saccharine (4.5 mg/tablet, 2% w/w). Next, a portion of mannitol was mixed with spearmint oil (0.5 mg/tablet, 0.22% w/w) and the mixture was sieved. The dry granulate containing the low substituted HPC, cetirizine HCl and Eudragit E100 was then mixed with the mixture of artificial cherry flavor, microcrystalline cellulose, sodium saccharine, mannitol and spearmint oil in a blender, the remaining portion of mannitol (total to 96 mg/tablet, 42.7% w/w) and magnesium stearate (2 mg/tablet, 0.89% w/w) were subsequently mixed in and the granulate was compressed into palatable tablets.

### Example 2

To cetirizine hydrochloride (5.0 mg/tablet, 2.2% w/w) dissolved in purified water (q.s.), PVP K-30 (12 mg/tablet, 5.3% w/w) was added. Crospovidone (50 mg/tablet, 22.2% w/w) was transferred to a high shear mixer, and the solution of cetirizine HCl and PVP K-30 was slowly added to the crospovidone while mixing to obtain an uniform granulate which was milled, and dried in a fluid bed dryer. Microcrystalline cellulose (60 mg/tablet, 26.7% w/w) was sieved with artificial cherry flavor (1 mg/tablet, 0.44% w/w) and saccharine sodium (4.5 mg/tablet, 2% w/w) and mixed in a blender. Next, a portion of mannitol was mixed with peppermint oil (0.5 mg/tablet, 0.22% w/w) and the mixture was sieved with the remaining portion of mannitol (total to 90 mg/tablet, 40% w/w). The dry granulate containing the PVP K-30, cetirizine HCl and crospovidone was mixed with the mixture of artificial cherry flavor, microcrystalline cellulose, sodium saccharine, mannitol and peppermint oil in a blender, magnesium stearate (2 mg/tablet, 0.89% w/w) was subsequently mixed in and the granulate was compressed into palatable tablets.

### Example 3

To cetirizine hydrochloride (5.0 mg/tablet, 2.2% w/w) and low substituted hydroxypropyl cellulose (50 mg/tablet, 22.2% w/w) were transferred to a high shear mixer and mixed. Eudragit E-100 (6 mg/tablet, 2.7% w/w) was dissolved in ethyl alcohol 95% (q.s.) and the resultant solution was added slowly to the high shear mixer while mixing to obtain an uniform granulate which was milled, dried in a fluid bed dryer and sieved. Artificial cherry flavor (1 mg/tablet, 0.44% w/w) was mixed with a portion of microcrystalline cellulose and then sieved with the remaining portion of microcrystalline cellulose (total to 60 mg/tablet, 26.7% w/w) and sodium saccharine (4.5 mg/tablet, 2% w/w). Next, a portion of mannitol was mixed with spearmint oil (0.5 mg/tablet, 0.22% w/w) and the mixture was sieved. The dry granulate containing the low substituted HPC, cetirizine HCl and Eudragit E 100 was then mixed with the mixture of artificial cherry flavor, microcrystalline cellulose, sodium saccharine, mannitol and spearmint oil in a blender, the remaining portion of mannitol (total to 96 mg/tablet, 42.7% w/w) and magnesium stearate (2 mg/tablet, 0.89% w/w) were subsequently mixed in and the granulate was compressed into palatable tablets.

### Results

The results for the stability of tablets prepared from Examples 1 and 2 are summarized in Tables 1 and 2. For comparison, the stability of the commercially available ZYRTEC^{®} tablet was also measured by high performance liquid chromatography (HPLC) and the results are also presented in Tables 1 and 2.

### HPLC - (a) assay determination

HPLC was performed on a 3 µm Hypersil Gold C18 column (250 x 4.6 mm). The mobile phase was a mixture of solutions A and B (60:40 v/v). Solution A was a solution of buffer and acetonitrile (75:25 v/v) and Solution B was a solution of buffer and acetonitrile (30:70 v/v), where the buffer was prepared by adding dihydrogen ammonium phosphate (55 g) in water (5000 ml), adding triethylamine (4 ml), and adjusting the pH to 2.5 with conc. H₃PO₄. The flow-rate was 1.0 ml/min and UV detector was set to 230 nm. The column temperature was 30°C, the diluent was water, and the injection volume was 50 µl.

The standard solutions were prepared as follows: a standard stock solution was prepared by accurately weighing cetirizine dihydrochloride (25 mg) A.S. into a 100 ml volumetric flask, adding acetonitrile (40 ml), and adding water to reach the total volume (conc. 0.25 mg/ml). A working standard solution was prepared by diluting 5 ml of the standard stock solution in a 250 ml volumetric flask and adding water to reach the total volume (conc. 0.005 mg/ml).

The sample solutions were prepared as follows: accurately weighing 10 tablets and calculating the average tablet weight. The accurately weighed (n) tablets were placed into a volumetric flask of volume (V) as shown in the following table:

| Strength (mg) | Amount of tablets (n) | Volume of the volumetric flask (V) |
|---|---|---|
| 5 | 5 | 100 |
| 10 | 5 | 200 |

Water was added either 60 ml for 5 mg/tablet dose or 120 ml of water for 10 mg/tablet dose, and the mixture was shaken for 30 minutes, sonicated for 10 minutes, and the final volume was reached by adding water. A portion of the solution (5.0 ml) was diluted in a 250 ml volumetric flask by adding water to reach the final volume. Thereafter, the solution was filtered through 0.45 µm PTFE filter disc (cetirizine conc. 0.005 mg/ml).

The system suitability test must conform to the following criteria: the typical retention time of the cetirizine peak is in the range of 4-7 minutes. The tailing factor of the cetirizine peak (calculated according to USP) should be not more than 2.0. The system suitability test was performed by injecting the system with the working standard solution, which was the suitability solution.

The procedure for assay determination was performed as follows: The diluent solution was injected to observe any system peaks. A sample of the relative standard solution was injected five times and the other standard solution was injected twice. For this study, the relative standard deviation of the cetirizine peak areas of the five injections should not be more than 2%. The relative difference between the averages of the normalized peak areas for the two standard solutions should be NMT 2%. Next, the sample solutions were injected into the HPLC, the chromatograms were run up to 1.5 times the retention time of the cetirizine peak and the peak area of cetirizine was determined for each chromatogram. After testing six injections of sample solution injection, the first standard solution was injected as a control.

The percent assay was calculated as follows. [Sample peak area response / average area std response] x [wt *std (mg)/100] x [5/250] x [sample volume (ml) / sample wt (mg)] x [average tablet wt (mg)/dosage unit (mg/tablet)] x 100 x DF** = % cetirizine of labeled amount
* Take into account the % water and the % assay of the standard.
* * DF = Dilution factor, for all dosage forms was 50.

### HPLC - (b) impurity and degradation products determination

The HPLC study was performed on a 3 µm Hypersil Gold C18 column (250 x 4.6 mm). The mobile phase was a mixture of solutions A and B as a gradient shown in the table below:

| Time (min) | Solution A (%) | Solution B (%) |
|---|---|---|
| 0 | 100 | 0 |
| 4 | 100 | 0 |
| 18 | 64 | 36 |
| 34 | 0 | 100 |
| 66 | 0 | 100 |
| 68 | 100 | 0 |
| 80 | 100 | 0 |

Solution A was prepared by adding triethylamine (2 ml) to water (800 ml), adjusting the pH to 7.5 with H₃PO₄ conc., and adding acetonitrile (200 ml). Solution B was prepared by adding triethylamine (2 ml) to water (200 ml), adjusting the pH to 7.5 with H₃PO₄ conc, and adding acetonitrile (800 ml). The flow-rate was 1.0 ml/min and UV detector was set to 230 nm. The column temperature was 26 °C, the diluent was water, and the injection volume was 50 µl.

The standard solutions were prepared as follows: a standard stock solution was prepared by accurately weighing cetirizine dihydrochloride (25 mg) A.S. into a 100 ml volumetric flask, adding acetonitrile (40 ml), and adding water to reach the final volume (conc. 0.25 mg/ml). A working standard solution was prepared by diluting 5 ml of standard stock solution in a 250 ml volumetric flask, using water to reach the final volume (conc. 0.005 mg/ml). The RL standard solution was prepared as follows: working standard solution (5 ml) was transferred into a 100 ml volumetric flask and water was added to reach the final volume (conc. 0.00025 mg/ml, 0.1% from sample solution).

The sample solutions were prepared as follows: accurately weighing 10 tablets and calculating the average tablet weight. Then accurately weighed (n) tablets into (V) ml volumetric flask according to the following table:

| Strength (mg) | Amount of tablets (n) | Volume of the volumetric flask (V) |
|---|---|---|
| 5 | 5 | 100 |
| 10 | 5 | 200 |

Water was added either 60 ml of for 5 mg/tablet dose or 120 ml for 10 mg/tablet dose, the mixture was shaken for 30 minutes, sonicated for 10 minutes, and water was added to reach the final volume. The solution was filtered through 0.45 µm PTFE filter disc (cetirizine conc. 0.25 mg/ml).

The system suitability test must conform to the following criteria: The typical retention time of the cetirizine peak is approximately 20-23 minutes. The tailing factor of the cetirizine peak (calculated according to USP) should be not more than 2.0. The main degradation peak elutes at RRT about 0.9 relative to cetirizine. The system suitability test was performed by injecting the system with a suitability solution. The system suitability solution was prepared as follows: 10 ml of stock standard solution was transferred (with 10 ml transparent volumetric flask) into SunTest system at 750 w/m²; 35°C for 45 minutes.

The procedure to determine impurity and degradation products was performed as follows: the diluent solution was injected to observe any system peaks, thereafter the (0.1%/RL) standard solution was injected. The integrator parameters were set to detect any peak area which was greater than the diluent solution. Thereafter, the working/IDD standard solution was injected six times, where the relative standard deviation of the six replicate injections of the IDD standard solution should be not more than 5%. Thereafter, the second standard solution was injected twice. The relative difference between averages of the normalized peak areas obtained for the two standard solutions should be NMT 10%. Next, sample solutions were injected into the chromatograph and the chromatograph was run for 80 minutes. The peak areas for each impurity were determined in each chromatogram. After injecting the sample solution four times, the first (working/IDD) standard solution was injected as a control.

The percent impurity/degradation product was calculated as follows. [Sample peak area / average area std response] x [wt *std (mg)/100] x [5/250] x [sample volume (ml) / sample wt (mg)] x [average tablet wt (mg)/dosage unit (mg/tablet)] x RRF] = % impurity/degradation product related to cetirizine. * Take into account the % water and the % assay of the standard.

| Name | RRF |
|---|---|
| Imp. A | 1.25 |
| Imp. 4-CBH | 1.73 |

### Relative retention time for know impurities/degradation products:

| I/DP Name | RRT |
|---|---|
| *Imp-A | 1.31 |
| *4-CBH | 1.38 |
| **Imp-B | 0.98 |
| **Imp-C | 0.94 |
| **Imp-D | 2.67 |
| **Imp-E | 1.01 |
| **Imp-F | 0.84 |
| **Ethyl Ester | 1.56 |

| | |
|---|---|
| *degradation product ** Process or synthetic impurity | |

The procedure for reporting was as follows: for any impurity/degradation product found at level < 0.1 % (RL) it was reported as < 0.1 %. For any known impurity/degradation product, the precise percentage was reported. For any other impurity/degradation product found at level above 0.1% (reporting limit), the precise percentage and RRT was reported. Report total (%) = Σ of all reported impurities. % RL (0.1%) = average area of IDD STD x RRF / 20

The results summarized in Tables 1-2 demonstrate that the tablet produced according to the present invention exhibits improved stability in comparison to the commercially available ZYRTEC^{®} tablet. The impurity levels found in the tablet made with the composition of the invention were considerably lower than those found in the commercially available ZYRTEC^{®} tablet after 3 months in storage at 40°C and a relative humidity of 75%.

**Table 1**

| Storage time | Test parameter | Tablet (5mg/tablet) prepared in Example 1 | Tablet (5mg/tablet) from Innovator (ZYRTEC^{®}) |
|---|---|---|---|
| 0 | Assay (%) | 99.9 | Not tested |
| | Total impurities/degradation products (%) | ND | Not tested |
| 3 months | Assay (%) | 101.9 | 101.89 |
| | Total impurities/degradation products (%) | 0.5 | 1.3 |

**Table 2**

| Storage time | Test parameter | Tablet (5mg/tablet) prepared in Example 2 | Tablet (5mg/tablet) from Innovator (ZYRTEC^{®}) |
|---|---|---|---|
| 0 | Assay (%) | 102.4 | Not tested |
| | Total impurities/degradation products (%) | 0.24 | Not tested |
| 3 months | Assay (%) | 111 | 101.89 |
| | Total impurities/degradation products (%) | 0.7 | 1.3 |

## Claims

1. A pharmaceutical composition comprising a granulate, wherein the granulate comprises a disagreeable-tasting drug, a filler and a binder, wherein the filler is low substituted hydroxyl propyl cellulose, crospovidone or microcrystalline cellulose, the binder is a polymethacrylate, polyvinylpyrrolidone, copovidone, cellulose derivatives, starch, dextrin or a combination thereof, wherein the pharmaceutical composition further comprises at least one extra granular excipient, and the pharmaceutical composition is not designed to be swallowed immediately.

2. A pharmaceutical composition according to Claim 1 in the form of an orally disintegrating or orally dispersible composition.

3. The pharmaceutical composition according to Claim 1 or Claim 2, wherein the pharmaceutical composition is retained in the oral or buccal cavity for approximately 5 to 50 seconds before being swallowed.

4. The pharmaceutical composition according to Claim 3, wherein the pharmaceutical composition is retained within the oral or buccal cavity for approximately of 5 to 10 seconds before being swallowed.

5. The pharmaceutical composition according to any preceding claim wherein the granulate consists essentially of a disagreeable-tasting drug, filler and binder.

6. The pharmaceutical composition according to any preceding claim wherein the filler is low substituted hydroxypropyl cellulose, or crospovidone.

7. The pharmaceutical composition according to any preceding claim wherein the filler is low substituted hydroxypropyl cellulose, or microcrystalline cellulose.

8. The pharmaceutical composition according to any preceding claim, wherein the filler is not used as ion exchange resin.

9. The pharmaceutical composition according to any preceding claim wherein the binder is polymethacrylate, polyvinylpyrrolidone, copovidone, starch or dextrin.

10. The pharmaceutical composition according to any preceding claim wherein the binder is polymethacrylate, polyvinylpyrrolidone, copovidone or starch.

11. The pharmaceutical composition according to any preceding claim wherein the binder is a polymethacrylate.

12. The pharmaceutical composition according to any preceding claim wherein the binder is a methyl methacrylate methacrylic acid copolymer.

13. A pharmaceutical composition according to any of Claims 1-5 comprising a granulate having a disagreeable-tasting drug, a filler and a binder, wherein the filler is low substituted hydroxypropyl cellulose, crospovidone or microcrystalline cellulose, the binder is a polymethacrylate, polyvinylpyrrolidone, copovidone, cellulose derivatives, starch, dextrin or a combination thereof, wherein the pharmaceutical composition further comprises at least one extra granular excipient, and wherein the pharmaceutical composition is a chewable tablet or oral dispersible tablet.

14. The pharmaceutical composition according to any preceding claim, wherein the disagreeable tasting drug is a substituted benzhydrylpiperazine non-sedating H₁-antihistamine.

15. The pharmaceutical composition according to any preceding claim wherein the disagreeable tasting drug is selected from the group consisting of cetirizine, efletirizine, buclizine, etodroxizine, hydroxyzine, and chlorcyclizine or an optically active isomer thereof, a hydrate thereof, or a pharmaceutically acceptable salt thereof, and preferably wherein the disagreeable tasting drug is cetirizine.

16. The pharmaceutical composition according to any preceding claim, wherein the pharmaceutical composition comprises a combination of two or more drugs, wherein at least one of the drugs has disagreeable taste.

17. The pharmaceutical composition according to any preceding claim, wherein the weight ratio between the filler and the disagreeable tasting drug is in a range between about 20:1 to about 3:1.

18. The pharmaceutical composition according to any preceding claim, wherein the weight ratio between the binder and the disagreeable tasting drug is in a range between 5:1 to about 1:1.

19. The pharmaceutical composition according to any preceding claim, wherein the composition is in the form of a chewable tablet or oral dispersible tablet.

20. The pharmaceutical composition according to any preceding claim, wherein the composition has less than about 1.4% impurities/degradation products after storage for 3 months at 40°C and a relative humidity of 75%.

21. A pharmaceutical composition according to any preceding claim wherein the disagreeable tasting drug is rendered palatable.

22. A process for preparing a pharmaceutical composition according to any preceding claim comprising dissolving a disagreeable-tasting drug and binder with a solvent to form a solution; admixing filler to the solution; removing the solution to obtain a mixture; and granulating the mixture.

23. A process according to Claim 22 further comprising adding one or more extragranular excipients to obtain a final blend.

24. A process according to Claim 22 or Claim 23 further comprising compressing the final blend into a solid dosage form.

25. The process according to claim 24, wherein the solid dosage form is a chewable tablet or oral dispersable tablet.

26. The process according to any of Claims 22 to 25, wherein the disagreeable tasting drug is a substituted benzhydrylpiperazine selected from the group consisting of cetirizine, efletirizine, buclizine, etodroxizine, hydroxyzine, and chlorcyclizine or an optically active isomer thereof, a hydrate thereof, and a pharmaceutically acceptable salt thereof.

27. A process according to Claim 26 wherein the disagreeable tasting drug is cetirizine.

28. The process according to any of Claims 22 to 27, wherein the binder is selected from the group consisting of pyrrolidone polymers and polymethacrylates.

29. The process according to any of Claims 22 to 28, wherein the weight ratio between the filler and the disagreeable tasting drug is in a range between about 20:1 to about 3: 1.

30. The process according to any of Claims 22 to 29, wherein the weight ratio between the binder and the disagreeable tasting drug is in a range between 5:1 to about 1:1.

31. The process according to any of Claims 22 to 30, wherein the granulate has less than about 1.4% to 0.005% impurities/degradation products of cetirizine after storage for 3 months at 40°C and a relative humidity of 75%.
